# EUROPEAN PATENT SPECIFICATION

(11) **EP 2 429 494 B1**
(45) Date of publication and mention of the grant of the patent: **17.07.2013**
(21) Application number: 10723504.6
(22) Date of filing: 10.05.2010
(51) Int. Cl.: A61K 31/616, A61K 47/48, A61K 9/14, A61K 47/18, A61K 9/19

(54) **PREPARATION OF ACETYL SALICYLIC ACID/GLUTAMIC ACID COMPLEX FOR ORAL ADMINISTRATION**
HERSTELLUNG EINES ACETYLSALICYLSÄURE-GLUTAMINSÄURE-KOMPLEXES ZUR ORALEN VERABREICHUNG
PRÉPARATION DE COMPLEXE ACIDE ACÉTYL SALICYLIQUE/ACIDE GLUTAMIQUE POUR UNE ADMINISTRATION ORALE

(30) Priority: 11.05.2009 SA 30028709
(43) Date of publication of application: 21.03.2012
(73) Proprietor: King Saud University, Riyadh 11451 (SA)
(72) Inventor: EHAB AHMED FOUAD AHMED, Riyadh 11451 (SA); FARS KAED M. ALANAZI, Riyadh 11451 (SA); MAHMOUD EL-BADRY ABDELMOTALEB ABDELRAHMAN, Riyadh 11451 (SA); IBRAHIM ABDULLAH IBRAHIM ALSARRA, Riyadh 11451 (SA)
(74) Representative: Goddar, Heinz J.
(86) International application number: PCT/EP2010/002866
(87) International publication number: WO 2010/130396

(56) References cited:
- DE-A1- 19 844 116
- GB-A- 1 525 765
- DATABASE WPI Week 200170 Thomson Scientific, London, GB; AN 2001-609110 XP002650277, OKABE, S.: "(2-Acetoxybenzoyl)glutamine, an aspirin derivative, its preparation, and antiinflammatory agents containing the derivative as the effective component", -& JP 2001 181251 A (NIPPON CHEMIPHAR CO) 3 July 2001 (2001-07-03)

## Description

The present invention refers to an ionic type complex of acetyl salicylic acid and L-glutamic acid. The present invention also refers to a method for preparing said complex. The present invention further refers to a pharmaceutical preparation comprising said complex for use in the prevention of side effects involved in acetylic salicylic acid administration.

### Background of the invention

Acetylsalicylic acid (Aspirin^{®}, ASA) is considered as one of the older analgesic antipyretic and non-steroidal anti-inflammatory drugs (NSAID), which is still used to date, especially for atherosclerosis patients as antiplatelet agent to prevent the formation of blood clots and to reduce the incidence of narrowing the coronary arteries. ASA is an acidic drug containing a free carboxylic group that induces a direct side effects causing inflammation of the tissues of the stomach if given by mouth, that may develop to peptic ulcers.

JP 2001/181251 discloses (2-acetoxybenzoyl)glutamine as an agent that alleviates gastric mucosa damage.

Therefore, it was an object of the present invention to provide a pharmaceutical preparation comprising ASA acid having less such side effects.

For the present invention the formation of a complex between ASA and glutamic acid was studied. Glutamic acid is an amino acid that can be obtained from foods and may form complexes with certain substances.

For a more detailed discussion of the object underlying the present invention is it referred to the Introduction of the publication of Ehab A. Fouad, Mahmoud El-Badry, Fars K. Alanazi, Maha M. Arafah, Ibrahim A. Alsarra, Peparation and Invetigation of Acetyl Salicylic Acid-Glutamic Acid Complex: A Novel Oral Delivery System. Digest Journal of Nanomaterials and Biostructures Vol. 4, No. 2, June 2009, p. 299-308,

### Summary of the invention

The object of the present invention is solved by an ionic type complex of acetyl salicylic acid and L-glutamic acid. Preferably, the molar ratio of acetylic salicylic acid and L-glutamic acid is 1:1.

In one embodiment, the complex is completely dissoluble in HCl, pH 1.2 within ten minutes.

The object of the present invention is further solved by a pharmaceutical preparation for oral administration comprising the complex of this invention.

In one embodiment, the pharmaceutical preparation is comprised by a solid dosage form.

The object of the present invention is further solved by the pharmaceutical preparation of this invention for use in the prevention or reduction of direct side effects of acetyl salicylic acid on stomach tissue, preferably gastric mucosa.

In one embodiment, acetyl salicylic acid is used as an analgetic, an antipyretic, a non-steroidal anti-inflammatory drug, or an antiplatelet agent.

In one embodiments, acetyl salicylic acid is used in the treatment of atherosclerosis, preferably to prevent the formation of blood clots and/or to reduce the incidence of narrowing coronary arteries.

In one embodiment, the pharmaceutical preparation of this invention is for long-term use of acetyl salicylic acid.

The object of the present invention is further solved by a method for preparing the complex of this invention or for preparing the pharmaceutical preparation of this invention comprising the step of freeze-drying.

In one embodiment, the method comprises the following steps:
(a) providing distilled water;
(b) adding and dissolving L-glutamic acid;
(c) adding and dissolving acetyl salicylic acid;
(d) freezing the solution obtained in step (c);
(e) freeze-drying the frozen solution obtained in step (d);
(f) collecting the dried powder obtained in step (e).

In one embodiment, the molar ratio of L-glutamic acid in step (b) and acetyl salicylic acid in step (c) is 1:1.

The idea forming a basis of the present invention was that studying the formation of a complex between ASA and glutamic acid and its preparation in the solid state may provide an easy handling for the preparation of solid dosage forms which may lead to a reduction in the direct side effects of ASA on the tissues of the stomach.

As a result, a novel ASA/glutamic acid complex has been successfully prepared using freeze-drying. Histologically, the complex did not result in any change or damage on rats' gastric mucosa after its oral administration. It is assumed that the ASA/glutamic acid complex will be a safe and a promising alternative to ASA for oral administration, in particular for long-term use of ASA.

### Detailed description of the invention

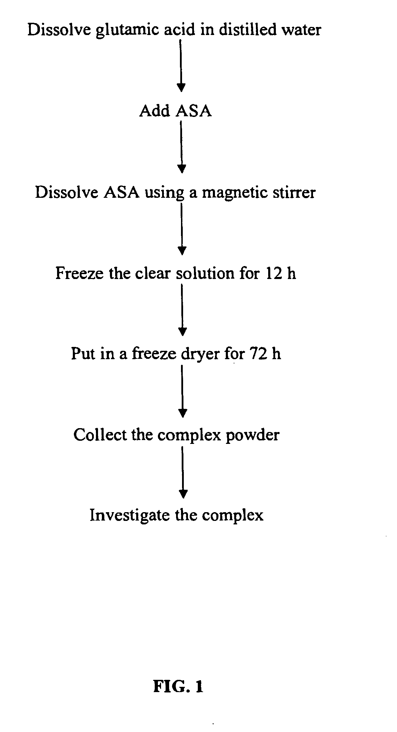

The present invention will be described in more detail with reference to Figures 1 to 7 as attached and Examples 1 to 9 below.
- Fig. 1:: is a schematic diagram illustrating the preparation of an ASA/glutamic acid complex.
- Fig. 2:: shows results obtained by differential scanning calorimetry (DSC) of ASA, L-gutamic acid and the formed complex.
- Fig. 3:: depicts scanning electron microscope micrographs showing the surface morphology of ASA (A), L-glutamic acid (B) and complex (C) crystals.
- Fig. 4:: shows a mass spectrum of the complex.
- Fig. 5:: shows a (Fourier transform infrared) FTIR chart for ASA, L-glutamic acid and the complex.
- Fig. 6: shows the morphology of gastric mucosa of rats after oral administration of ASA/glutamic acid complex solutions (x200). Control rat stomach (A), rat stomach after complex administration (B).
- Fig. 7: shows the dissolution of ASA and ASA/glutamic acid complex in HCl, pH 1.2.

### EXAMPLES

It is also referred to the Experimental section and the Results and Discussion of the aforementioned publication of Foud et al. (2009) Digest Journal of Nanomaterials and Biostructures: 4, 299-308.

### EXAMPLE 1: Preparation of ASA/glutamic acid complex

Equimolar (0.01 mole) weights of ASA and L-glutamic acid were accurately weighed. L-glutamic acid was dissolved in 400 ml distilled water (HPLC grade). Then ASA was added and dissolved in the same vessel. The prepared solution was allowed to freeze overnight at (-20°C) and then lyophilized over a period of 72 hrs using a freeze-drier. The dried powder was collected and stored at room temperature in a desiccator until further investigations.

### EXAMPLE 2: Differential scanning calorimetry

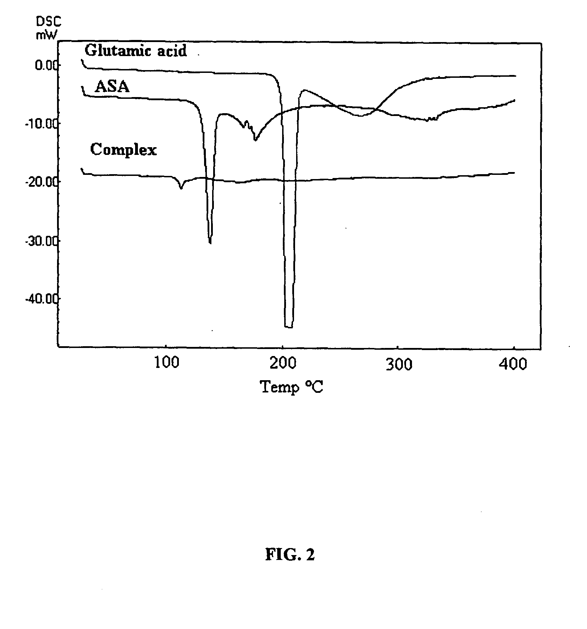

The thermal behavior for each of ASA, L-glutamic acid and their complex was studied using the differential scanning calorimetry (DSC) and presented in Figure 2 and Table 1. The data in the thermogram of ASA showed a characteristic sharp endothermic peak at 138.02°C corresponding to its melting point and ΔH 194.03 mj/g. However the thermogram of L-glutamic acid gives a sharp peak at 207.51 °C also corresponding to its melting point and ΔH 496.72 mj/g. The freeze dried powder showed that an interaction conducted between the two compounds giving a new small endothermic peak having a lower melting point, 113.3 °C, and lower heat, ΔH 7.47 mj/g. At the same time the peaks of ASA and L-glutamic acid were completely disappeared. The present result demonstrates the formation of a complex between ASA and L-glutamic acid in water that can be collected by a freeze drying method.

**Table 1: Peak temperature and enthalpy (ΔH) obtained from DSC thermogram.**

| Parameter | ASA | L-Glutamic acid | Complex |
|---|---|---|---|
| Peak °C | 138.02 | 207.51 | 113.3 |
| Heat (ΔH) | - 970.16 mj | - 2.48 j | - 37.35 mj |
| Heat mj/g | - 194.03 | - 496.72 | -7.47 |

### EXAMPLE 3: Scanning electron microscopy

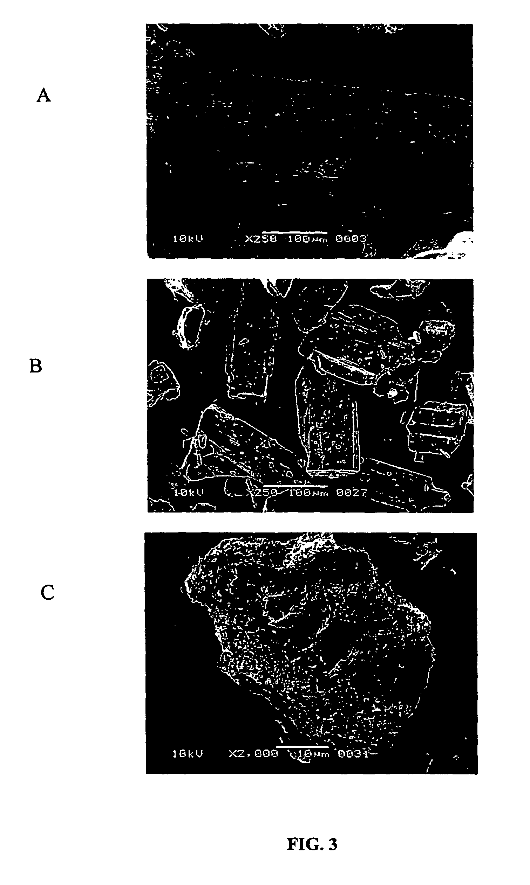

Figure 3 shows the scanning electron microscope (SEM) photographs of the used ASA, L-glutamic acid and the resulted complex. The morphological shape of the formed complex crystals showed a clear difference in shape. These crystals of the formed complex appear as an aggregation of flakes to form a snail like structure of a rough surface with holes. However, ASA crystals are needle-like shaped and those of L-glutamic acid are large orthorhombic shaped particles.

### EXAMPLE 4: Mass spectrometry study

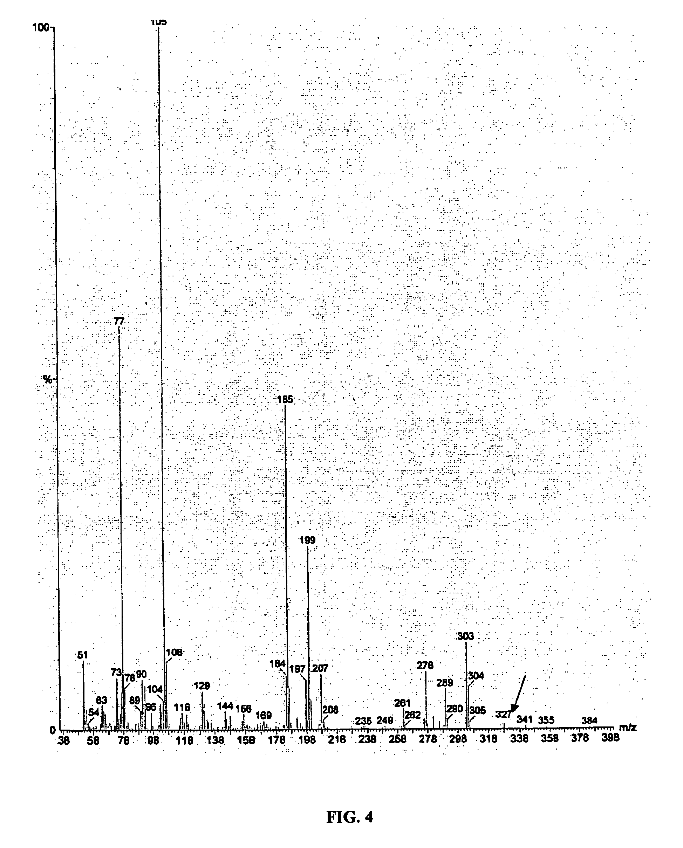

As the molecular weight of ASA is 180 and that of L-glutamic acid is 147, the expected molecular weight of the complex is the sum of the two molecular weights i.e 327. Mass spectrum showed a small peak at 327 representing the molecular ion peak which reflected the formation of a complex with a stoichiometric ratio 1:1. (Fig. 4)

### EXAMPLE 5: FTIR spectroscopy study

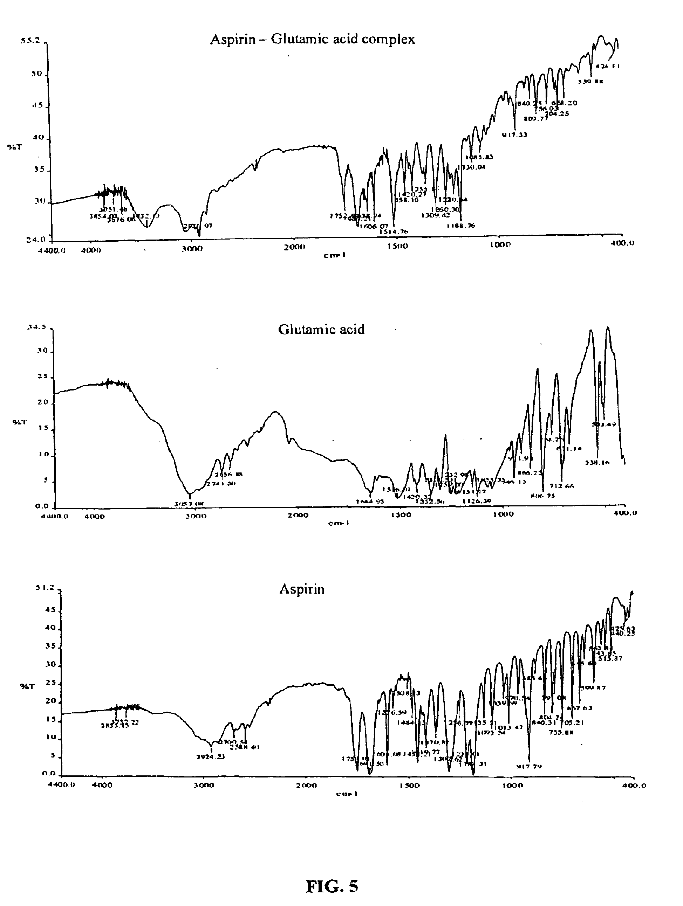

Figure 5 shows the Fourier transform infrared (FTIR) spectrum of ASA, glutamic acid and the formed complex, while Table 2 presents the bands frequencies of each of ASA and glutamic acid that are affected as a result of their interaction.

As glutamic acid is an amino acid characterized by the presence of an amino group and two carboxylic groups, it was noticed that the stretching and bending bands of the quaternary nitrogen bands wave numbers were decreased reflecting electron donation from the NH₃⁺. Consequently, there was an increase in the wave number of the stretching band of C-N from 1352 to 1356 cm⁻¹ reflecting an electron withdrawing effect. However, the bands of carboxylic groups were not affected. On the other hand, the effect of the interaction on the FTIR spectrum of ASA was noticed through the band of the C=O of the carboxylic group, where it was slightly shifted towards lower frequency value from 1690 to 1686 cm⁻¹. At the same time, the bands of C=O of the aryl ester of ASA did not show any changes in its wave numbers. This data may reflect an interaction between the carboxylate and the quaternary nitrogen.

**Table 2: FTIR bands wave number (cm⁻¹) affected by complex formation**

| ASA | Glutamic acid | | |
|---|---|---|---|
| 1690 | 3075 | 1516 | 1352 |

| Complex | | | |
|---|---|---|---|
| 1686 | 3061 | 1514 | 1356 |

### EXAMPLE 6: Nuclear magnetic resonance studies

Nuclear magnetic resonance (¹H NMR) spectra showed the following:

ASA (DMSO-d₆): 2.25 (3H, d, j=5 Hz, CH₃C=O), 7.19 (1H, s, C₃-H), 7.36 (1H, d, j=6 Hz, C₅-H), 7.61 (1H, d, j=6 Hz, C₄-H), 7.96 (1H, d, j= 6 Hz, C₆-H), 13.12 (1H, s, COOH). ASA-Glutamic acid complex (DMSO-d₆): 2.25 (3H, s, CH₃C=O), 7.2 (1H, d, j=8 Hz, C₃-H), 7.38 (1H, t,j=15,7.5 Hz, C₅-H), 7.64 (1H, m, C₄-H), 7.39 (1H, m, C₆-H). L-Glutamic acid (D₂O): 2.09 (2H, m, C₃-H₂), 2.5 (2H, m, C₄-H₂), 3.76 (1H, m, C₂-H). ASA-Glutamic acid complex (D₂O): 2.04 (3H, m, CH₃C=O), 2.11(2H, m, C₃-H₂), 2.33 (2H, m, C₄-H₂), 3.8 (1H, m, C₂-H), 6.96 (1H, m, C₃-H), 7.17 (1H, m, C₅-H), 7.49 (1H, m, C₄-H ), 7.88 (1H, m, C₆-H).

Studying the ¹H NMR of ASA and the effect of glutamic acid on its ¹H NMR, each of ASA and complex were dissolved in DMSO. The results show the following:
(1) The protons of the acetyl group did not show any change in their behavior.
(2) The aromatic protons at carbons number 3, 4 and 5 showed a down field shift, while the proton at carbon number 6 showed an upper field shift. All these chemical shifts lie between 0.01 and 0.03 ppm which appear insignificant.
(3) The carboxylic acid proton at 13.12 ppm disappeared in the chart of the complex.
(4) The glutamic acid protons were detected but they were not integrated in unexplained phenomenon.

For testing the effect of ASA on the glutamic acid each of glutamic acid and the complex were run in D₂O. The results show the following:
(1) The appearance of two protons around 2.09 ppm of C₃ which was insignificantly up-field shifted to 2.11 ppm in case of the complex.
(2) The two protons at C4 and the one at C2 showed up-field shifting by 0.17 and 0.04 ppm, respectively.

The ¹HNMR results indicated the complex formation, however, the contributed groups can not be explained or estimated yet.

Accordingly, the given spectral data indicated the formation of a complex between ASA and glutamic acid in 1:1 molar ratio. This complex, most probably, can be described as ionic type complex involving both the carboxylic and the amino function groups of ASA and glutamic acid, respectively.

### EXMAPLE 7: Histopathology study

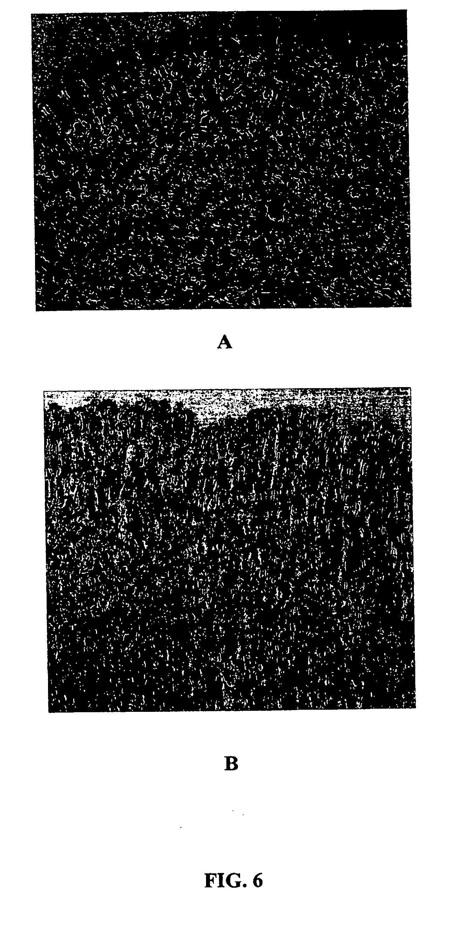

The effect of the complex on the gastric mucosa was studied using Wistar rats. As the aim of this work was to decrease the effect of ASA on stomach tissues, so, the ASA/glutamic acid complex was administered orally to male Wistar rats and the histological changes are shown in Figure 6. It was found that the administration of the ASA/glutamic acid complex had no dramatic effect on the gastric mucosa as it appeared normal without any changes.

### EXAMPLE 8: In vitro dissolution study

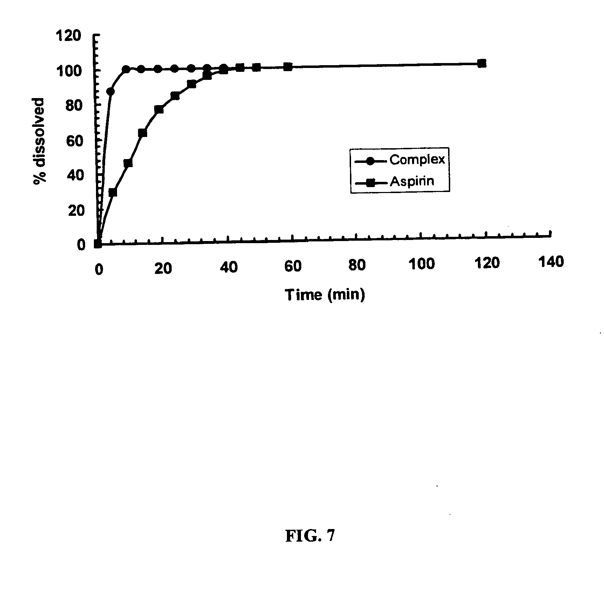

Each of ASA and the complex was allowed to dissolve in HCl, pH 1.2, as presented in Figure 7. ASA was found to completely dissolve between 40 and 45 min. However, the complex was completely soluble within ten minutes.

## Claims

1. An ionic type complex of acetyl salicylic acid and L-glutamic acid at a molar ratio of 1:1.

2. The complex according to claim 1, which complex is dissolved in HCl, pH 1.2 within ten minutes.

3. A pharmaceutical preparation for oral administration comprising the complex according to claim 1 or 2.

4. The pharmaceutical preparation according to claim 3, which preparation is comprised by a solid dosage form.

5. A pharmaceutical preparation according to claim 3 or 4, for use in the prevention or reduction of side effects of acetyl salicylic acid on stomach tissue, preferably gastric mucosa.

6. The pharmaceutical preparation according to claim 5, wherein acetyl salicylic acid is used as an analgetic, an antipyretic, a non-steroidal anti-inflammatory drug, or an antiplatelet agent.

7. The pharmaceutical preparation according to claim 5, wherein acetyl salicylic acid is used in the treatment of atherosclerosis, preferably to prevent the formation of blood clots and/or to reduce the incidence of narrowing the coronary arteries.

8. The pharmaceutical preparation according to any of claims 5 to 7, for long-term use of acetyl salicylic acid.

9. A method for preparing the complex according to claim 1 or 2, or for preparing the pharmaceutical preparation according to claim 3 or 4, comprising the step of freeze-drying.

10. The method according to claim 9 comprising the following steps:
(a) providing distilled water;
(b) adding and dissolving L-glutamic acid;
(c) adding and dissolving acetyl salicylic acid;
(d) freezing the solution obtained in step (c);
(e) freeze-drying the frozen solution obtained in step (d);
(f) collecting the dried powder obtained in step (e).

11. The method according to claim 9 or 10, wherein the amounts of L-glutamic acid in step (b) and of acetyl salicylic acid in step (c) are equimolar.

## Patentansprüche

1. Ionen-Komplex aus Acetylsalicylsäure und L-Glutaminsäure in einem Molverhältnis von 1:1.

2. Komplex nach Anspruch 1, wobei dieser Komplex in HCl, pH 1,2, innerhalb von zehn Minuten gelöst wird.

3. Pharmazeutische Zubereitung für orale Verabreichung, die den Komplex nach Anspruch 1 oder 2 umfasst.

4. Pharmazeutische Zubereitung nach Anspruch 3, wobei diese Zubereitung von einer festen Dosierungsform umfasst ist.

5. Pharmazeutische Zubereitung nach Anspruch 3 oder 4 zur Verwendung bei der Verhinderung oder Verringerung von Nebenwirkungen von Acetylsalicyclsäure auf Magengewebe, insbesondere Magenschleimhaut.

6. Pharmazeutische Zubereitung nach Anspruch 5, wobei Acetylsalicylsäure als ein Analgetikum, eine Antipyretikum, ein nicht-steroidaler entzündungshemmender Arzneistoff oder ein Antithrombozytenmittel verwendet wird.

7. Pharmazeutische Zubereitung nach Anspruch 5, wobei Acetylsalicylsäure bei der Behandlung von Atherosklerose verwendet wird, vorzugsweise um die Bildung von Blutgerinnseln zu verhindern und/oder das Auftreten einer Verengung der Koronararterien zu verringern.

8. Pharmazeutische Zubereitung nach einem Ansprüche 5 bis 7 zum Langzeitgebrauch von Acetylsalicylsäure.

9. Verfahren zur Herstellung des Komplexes nach Anspruch 1 oder 2 oder zur Herstellung der pharmazeutischen Zubereitung nach Anspruch 3 oder 4, umfassend den Schritt der Gefriertrocknung.

10. Verfahren nach Anspruch 9, umfassend die folgenden Schritte:
(a) Bereitstellen von destilliertem Wasser;
(b) Zugeben und Lösen von L-Glutaminsäure;
(c) Zugeben und Lösen von Acetylsalicylsäure;
(d) Einfrieren der in Schritt (c) erhaltenen Lösung;
(e) Gefriertrocknen der in Schritt (d) erhaltenen gefrorenen Lösung;
(f) Sammeln des in Schritt (e) erhaltenen getrockneten Pulvers.

11. Verfahren nach Anspruch 9 oder 10, wobei die Mengen an L-Glutaminsäure in Schritt (b) und an Acetylsalicylsäure in Schritt (c) äquimolar sind.

## Revendications

1. Complexe de type ionique d'acide acétylsalicylique et d'acide L-glutamique à un rapport molaire de 1:1.

2. Complexe selon la revendication 1, lequel complexe est dissout dans du HCl, pH 1,2 pendant dix minutes.

3. Préparation pharmaceutique pour administration par voie orale comprenant le complexe selon la revendication 1 ou 2.

4. Préparation pharmaceutique selon la revendication 3, laquelle préparation est constituée d'une forme galénique solide.

5. Préparation pharmaceutique selon la revendication 3 ou 4, destinée à être utilisée dans la prévention ou la réduction des effets secondaires de l'acide acétylsalicylique sur le tissu gastrique, de préférence sur la muqueuse gastrique.

6. Préparation pharmaceutique selon la revendication 5, dans laquelle l'acide acétylsalicylique est utilisé en tant que médicament analgésique, antipyrétique, anti-inflammatoire non-stéroïdien, ou un agent antiplaquettaire.

7. Préparation pharmaceutique selon la revendication 5, dans laquelle l'acide acétylsalicylique est utilisé dans le traitement de l'athérosclérose, de préférence pour prévenir la formation de caillots de sang et/ou pour réduire l'incidence de rétrécissement des artères coronaires.

8. Préparation pharmaceutique selon l'une des revendications 5 à 7, pour une utilisation à long terme de l'acide acétylsalicylique.

9. Procédé de préparation du complexe selon la revendication 1 ou 2, ou de la préparation pharmaceutique selon la revendication 3 ou 4, comprenant l'étape de lyophilisation.

10. Procédé selon la revendication 9, comprenant les étapes suivantes consistant :
(a) à fournir de l'eau distillée ;
(b) à ajouter et à dissoudre l'acide L-glutamique ;
(c) à ajouter et à dissoudre l'acide acétylsalicylique ;
(d) à congeler la solution obtenue dans l'étape (c) ;
(e) à lyophiliser la solution congelée obtenue dans l'étape (d) ;
(f) à collecter la poudre séchée obtenue dans l'étape (e).

11. Procédé selon la revendication 9 ou 10, dans lequel les quantités de l'acide L-glutamique dans l'étape (b) et de l'acide acétylsalicylique dans l'étape (c) sont équimolaires.
